# EUROPEAN PATENT SPECIFICATION

(11) **EP 2 640 282 B1**
(45) Date of publication and mention of the grant of the patent: **28.05.2014**
(21) Application number: 11782666.9
(22) Date of filing: 08.11.2011
(51) Int. Cl.: A61B 17/15

(54) **SURGICAL INSTRUMENT SYSTEM, SURGICAL ALIGNMENT GUIDE AND SURGICAL ROD**
CHIRURGISCHES INSTRUMENTENSYSTEM, CHIRURGISCHE AUSRICHTUNGSFÜHRUNG UND CHIRURGISCHER MARKNAGEL
SYSTÈME D'INSTRUMENTS CHIRURGICALS, GUIDE D'ALIGNEMENT CHIRURGICAL ET TIGE CHIRURGICAL

(30) Priority: 18.11.2010 GB 201019492
(43) Date of publication of application: 25.09.2013
(73) Proprietor: DePuy (Ireland), Cork, County Cork (IE)
(72) Inventor: BOOTH, Kevin, Leeds Yorkshire LS11 8DT (GB); FENTON, Gary, Leeds Yorkshire LS11 8DT (GB); VERTERAMO, Alberto, Leeds Yorkshire LS11 8DT (GB)
(74) Representative: Belcher, Simon James
(86) International application number: PCT/GB2011/052172
(87) International publication number: WO 2012/066305

(56) References cited:
- EP-A1- 0 687 448
- WO-A2-2009/037471
- US-A- 5 683 396
- US-A- 5 688 281
- US-A1- 2004 153 084
- US-A1- 2009 043 309
- US-A1- 2010 234 850

## Description

The present invention relates to a surgical alignment guide and a surgical rod for use with the surgical alignment guide. Together the surgical alignment guide and surgical rod form a surgical instrument system. The present invention is particularly applied to orthopaedic surgery and especially knee surgery.

In orthopaedic knee surgery, a cut may be made to the femoral head in order to correct varus or valgus alignment. A cutting guide is used to locate the cut accurately. The cutting guide is fixed to the bone using pins and provides a stable surface to guide resection of the femoral head.

In order to ensure the cutting guide is placed correctly on the femur, an alignment system is typically used. An intramedullary rod is inserted into the intramedullary canal of the femur, providing a stable reference to the intramedullary axis of the femur. An alignment guide is disposed on this rod. The alignment guide contains a scale indicating the desired angle of the cut relative to the intramedullary axis of the femur. The cutting guide is attached to the alignment guide and advanced along the rod until it is in contact with the femur, where it is aligned at the desired angle by the alignment guide. It can then be secured in place.

During insertion of the intramedullary rod to the intramedullary canal, a surgeon will typically use two hands for the procedure: one to secure the alignment guide relative to the rod and one to insert the rod. It would be desirable if insertion of the rod could be a one handed operation.

US-A-2010/234850 discloses a surgical instrument assembly for use in a procedure to implant a knee joint prosthesis, having the features specified in the pre-characterising part of claim 1.

The present invention provides a system of intramedullary rod and alignment guide which comprise a restraining system to maintain the relative longitudinal and rotational position of an alignment guide relative to a rod, while the rod is inserted into the intramedullary canal.

Accordingly the invention provides a surgical instrument system as defined in claim 1,

In use, the alignment guide is for aligning the cutting guide. The rod is for insertion into the intramedullary canal to locate the alignment guide relative to the intramedullary axis of a bone for accurate location of a cutting guide against the bone. The alignment guide is normally unrestrained relative to the rod and free to move along the rod. The restraining system restrains the alignment guide towards the proximal end of the rod and enables insertion and removal of the rod to be carried out with one hand, unlike prior art methods which required holding the alignment guide to secure it relative to the rod during insertion and removal. At least a portion of the restraining system is permanently located towards the proximal end of the rod.

The restraining system includes a resilient element and recess configured to engage the resilient element. Such a construction can provide secure restraint of the alignment guide while also being easy to engage and release. The resilient element may be located on either the rod or the alignment and the recess is then located on the other of the rod and the alignment guide.

The restraining system may comprise a resilient member disposed on the surface of the rod. Preferably, the resilient member is disposed on the surface of the cylindrical member. It can be ring shaped, or at least partially ring shaped and may be located towards the proximal end of the cylindrical member. This ensures the alignment guide is restrained sufficiently proximal to the distal end of the cylindrical section and that it does not interfere with insertion or removal of the rod.

The restraining system may further comprise a circumferential recess formed in the through bore of the alignment guide engaging the resilient member. The circumferential recess may extend partially or completely around the circumference of the through bore. This can provide a more secure connection.

In some embodiments the restraining system may be further for preventing rotation of the alignment guide relative to the rod about the longitudinal axis. Although some prevention of rotation may be provided by the friction between the resilient member and the through bore of the alignment guide, this may not be sufficient to prevent rotation of the alignment guide. Adapting the restraining system to specifically prevent rotational motion reduces the risk of relative rotation between the alignment guide and the rod.

In one embodiment, the restraining system may comprise a protrusion on the rod for engaging a corresponding recess in the alignment guide. This engagement can prevent rotation. For example, the protrusion may be a polygon with a constant cross-section along the longitudinal axis. More preferably, the polygon has rotational symmetry about the longitudinal axis. For example, the polygon may be a regular polygon with its centre on the longitudinal axis. The rotational symmetry can allow more freedom in the relative rotational alignment of the alignment guide to the rod when the restraining system is engaged. Regular polygons include hexagons, octagons, etc. Alternatively, other polygonal shapes with rotational symmetry may be used.

The surgical alignment guide can comprise:
a body section having a through bore, wherein the through bore defines a longitudinal axis for receiving a rod, and
a rod retaining element; wherein the rod retaining element is for engaging part of the rod, when the rod is located in the through bore.

The rod retaining element prevents relative movement of the alignment guide along a rod when the alignment guide is installed on a rod.

The rod retaining element may comprise a circumferential recess extending around a circumference of the through bore. This recess may have a greater dimension perpendicular to the longitudinal axis than the through bore, i.e. it may have a greater diameter than the through bore. This can engage a corresponding protrusion on the rod. Alternatively, the rod retaining element may be a protrusion extending into the through bore. The rod retaining element may comprise a resilient element extending into the through bore.

The alignment guide may further comprise a longitudinal recess for engaging a corresponding protrusion on the rod in order to prevent rotation about the longitudinal axis. This may comprise a channel with surfaces defining at least parts of a polygon with constant cross-section along the longitudinal axis. Preferably, the channel comprises surfaces defining at least part of a polygon having rotational symmetry about the longitudinal axis.

The intramedullary rod can comprise a cylindrical member defining a longitudinal axis, and an alignment guide retaining element located towards a proximal end of the rod. The alignment guide retaining element prevents movement of an alignment guide along longitudinal axis when an alignment guide is inserted onto the cylindrical member. Its location at the proximal end ensures that the alignment guide is retained a way from the distal end of the cylindrical section which is inserted into the intermedullary canal.

The alignment guide retaining element may be resilient and extend around at least a portion of the circumference of the cylindrical member. It may have a diameter greater than the diameter of the cylindrical member. This can engage a corresponding feature on the alignment guide to provide a secure connection.

The alignment guide retaining element may comprise a circumferential recess extending around at least a portion of the cylindrical member.

The intramedullary rod may further comprise a protrusion for engaging a corresponding recess on the alignment guide, so that the engagement prevents rotation about the longitudinal axis. In one embodiment the protrusion comprises a polygon with a constant cross-section along the longitudinal axis, preferably the polygon has rotational symmetry about the longitudinal axis.

Embodiments of the invention will now be explained by way of example and not limitation with reference to the accompanying drawings, in which:
Figure 1 depicts a perspective view of an alignment guide, cutting guide and intramedullary rod before connection of the cutting guide to the alignment guide;
Figure 2 depicts a perspective view of an alignment guide, cutting guide and intramedullary rod as shown in Figure 1, after the cutting guide has been connected to the alignment guide.
Figure 3 shows a plan view of the system of alignment guide, cutting guide and rod of Figure 2;
Figure 4 depicts a cross-section through the attachment protrusion and corresponding recess when the alignment guide has been connected to the cutting guide;
Figures 5a-5e show cross-sections through the alignment guide and cutting guide showing the disconnection of the alignment guide from the cutting guide;
Figure 6 depicts an intramedullary rod for use with the cutting guide and alignment guide of the system;
Figure 7 depicts a perspective view of an alignment guide installed on the intramedullary rod of Figure 6;
Figure 8 depicts a cross-section through the intramedullary rod and alignment guide before the alignment guide is secured on the intramedullary rod;
Figure 9 depicts a cross-section through the intramedullary rod and alignment guide after the alignment guide is secured on the intramedullary rod;
Figure 10 depicts a cross-section showing a rotation limiting connection between the alignment guide and the intramedullary rod;
Figure 11 depicts a perspective view of an alternative alignment guide;
Figure 12 depicts a cross section of the alignment guide of Figure 11;
Figure 13 depicts an exploded view of selected components of the alignment guide of Figure 11;
Figure 14 depicts a perspective view of an adjustment member of the alignment guide of Figure 13;
Figure 15 depicts an end view of the adjustment member of Figure 14;
Figures 16A-16C depict cross sections through the adjustment member of Figure 14;
Figure 17 depicts a perspective view of a pivoting member of the alignment guide of Figure 13;
Figure 18 depicts an exploded view of a cutting guide attachment part for use with the alignment guide of Figure 16; and
Figure 19 depicts a perspective view of an alignment guide, cutting guide and intramedullary rod with the intramedullary rod inserted to the intramedullary canal of a femur.

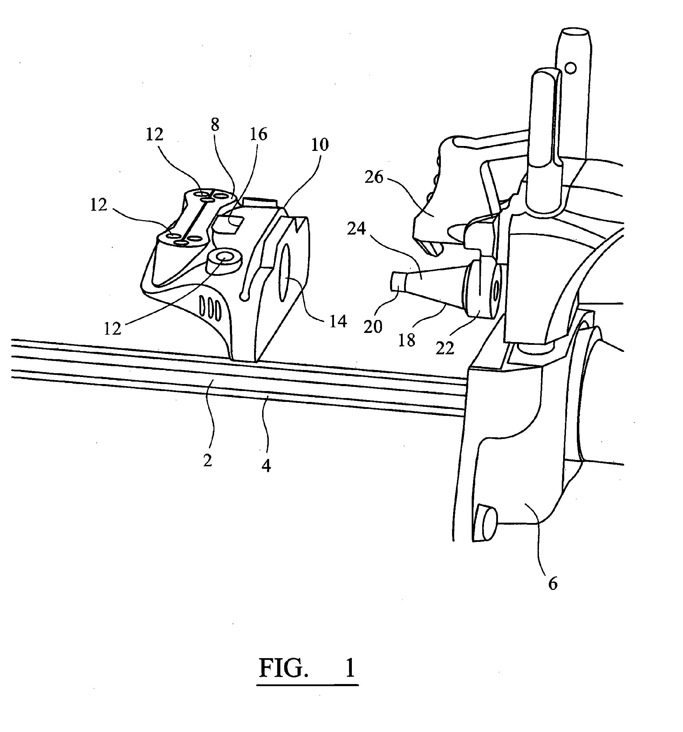

Figure 1 depicts a perspective view showing components of a surgical instrument system for aligning a cutting guide. The system comprises an intramedullary rod which comprises a cylindrical member 2 having longitudinal grooves 4 formed along its length. In use the cylindrical section 2 is inserted into the intramedullary canal of a femur and the longitudinal grooves 4 provide means for pressure release during insertion.

An alignment guide 6 (shown partially in Figure 1) comprises a through bore into which the cylindrical section 2 of the intramedullary rod is inserted, so that the alignment guide 6 can move longitudinally along the cylindrical section 2 and also rotate relative to the cylindrical section.

A cutting guide 8 is provided separately from the alignment guide 6. The cutting guide 8 comprises a cutting slot 10 which defines the cut to be made to the bone. Cutting guide 8 also comprises attachment holes 12 for fixing the cutting guide 8 to the bone. Cutting guide 8 is attached to the alignment guide 6 by means of a recess 14 and an attachment surface 16 on its upper surface. The recess 14 has a shape corresponding to an attachment protrusion 18 formed on the alignment guide 6.

Attachment protrusion 18 comprises a first cylindrical portion 20 having a first diameter and a second cylindrical portion 22 having a second diameter which is larger than the first diameter. The first diameter is about 4mm and the second diameter is about 12mm in this embodiment. Other dimensions may be used in other embodiments. The first and second cylindrical portions 20, 22 share a common axis. Joining the first cylindrical section to the second cylindrical section 22 is a generally frustoconical portion 24. The recess 14 defines surfaces corresponding to the first and second cylindrical portions 20, 22 of the attachment protrusion 18.

In use, when the attachment protrusion 18 is inserted into recess 14, first cylindrical portion 20 and second cylindrical portion 22 engage corresponding surfaces within the recess so that the cutting guide 8 is securely aligned with the longitudinal axis of the attachment protrusion 18. The dimensions of the corresponding surfaces within the recess are close to the dimensions of the first cylindrical portion 20 and the second cylindrical portion 22 but very slightly larger. This ensures firm connection but reduces the likelihood of a tight fit between the attachment protrusion 18 and the recess 14 making it difficult to remove the attachment protrusion 18 from the recess 14.

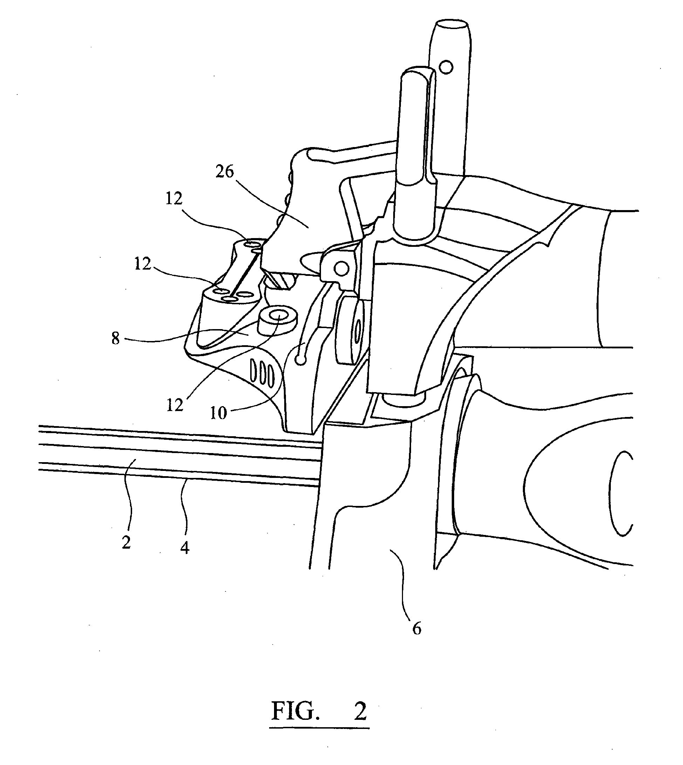

The cutting guide 8 is further secured in place on the attachment protrusion 18 of the alignment guide 6 by a clip member 26 on the alignment guide 6. The clip member 26 engages the attachment surface 16 of the cutting guide 8. A perspective view of the cutting guide 8 installed on the alignment guide 6 can be seen in Figure 2.

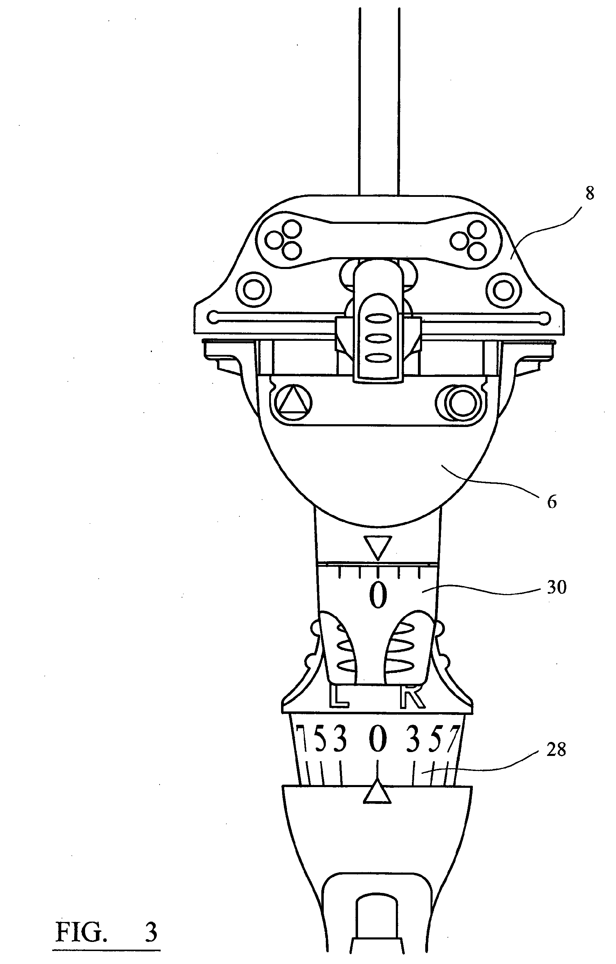

Figure 3 shows a plan view of the system of alignment guide 6, cutting guide 8 and rod assembled together. Figure 3 also depicts two adjustment scales 28, 30 provided on the alignment guide 6. The two adjustment scales 28, 30 allow the relative rotation of parts of the alignment guide to be set as determined by a surgeon to alter the varus valgus rotation of the attachment protrusion 18 relative to the intramedullary axis defined by the cylindrical portion 2 of the rod and the through bore in the alignment guide 6. The cutting guide 8 is installed on the attachment protrusion 18 and hence its alignment is altered relative to the intramedullary axis.

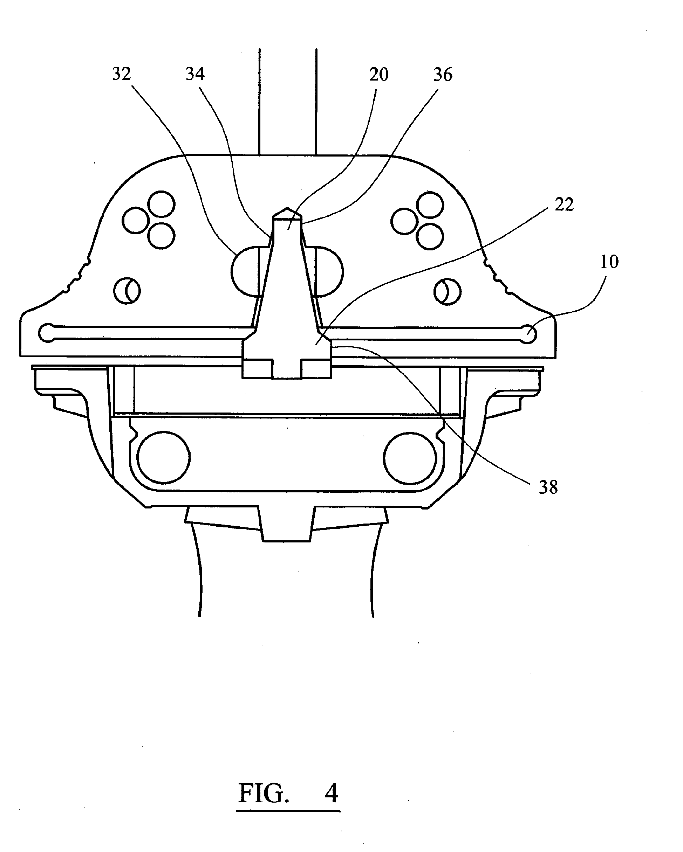

Figure 4 depicts a cross-section of the assembled system, taken through the connection recess. This shows how the cutting guide is secured on the alignment guide by the engagement of cylindrical portions 20, 22 in corresponding sections of the recess. Figure 4 also enables the internal construction of the connection recess 14 to be understood more clearly. The cross-section illustrates enlarged portion 32. Enlarged portion 32 includes an initial section perpendicular to the longitudinal axis of the recess following a short tapered section 34 from the first cylindrical section 36. Enlarged section 32 has a greatest dimension perpendicular to the longitudinal axis which is larger than the diameter of the second cylindrical section 38. This provides a greater range of movement for the first cylindrical section 20 within the recess during disconnection of the alignment guide 6 from the cutting guide 8. Tapered section 34, adjacent the first cylindrical section 36 of the cutting guide 8 serves to guide the tip of the attachment protrusion 18 into the first cylindrical section 36.

The enlarged central section 32 extends perpendicular to the longitudinal axis through the entire depth of the cutting guide. This allows the enlarged central section to also provide attachment surface 16 for clip 26.

The second cylindrical section 38 is formed in the portion of the cutting guide adjacent cutting slot 10.

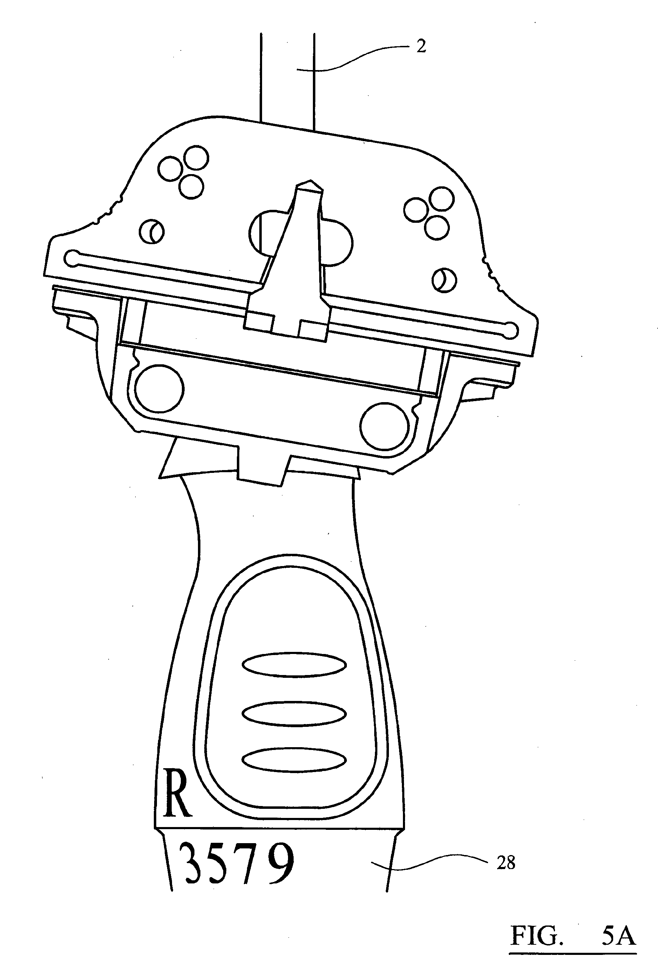

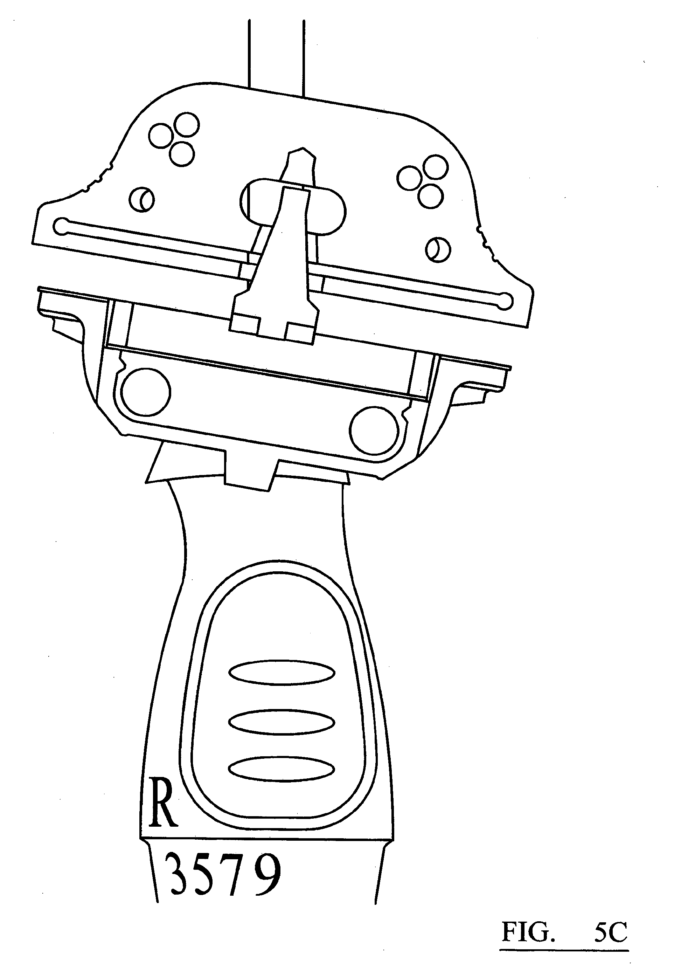

The disconnection of the cutting guide from the alignment guide will now be described. To illustrate the benefits of this system, the cutting guide is depicted in Figure 5a connected to the alignment guide 6 with the varus valgus adjustment 28 of the alignment guide adjusted to a maximum in the right-hand direction. This shifts the angle of the longitudinal axis of the attachment protrusion 18 and cutting guide 8 relative to the longitudinal axis of the cylindrical section 2, and can clearly be seen in Figure 5a. In prior art devices this configuration can be difficult for a surgeon to disconnect the alignment guide from the cutting guide. The difference in angles between the anatomic axis (defined by the cylindrical section 2 of the rod) and the mechanical axis, in addition to the offset of the attachment protrusion with the alignment guide make it difficult to remove cleanly. The alignment guide is constrained by the cylindrical section 2 to move along the anatomical axis, not mechanical axis.

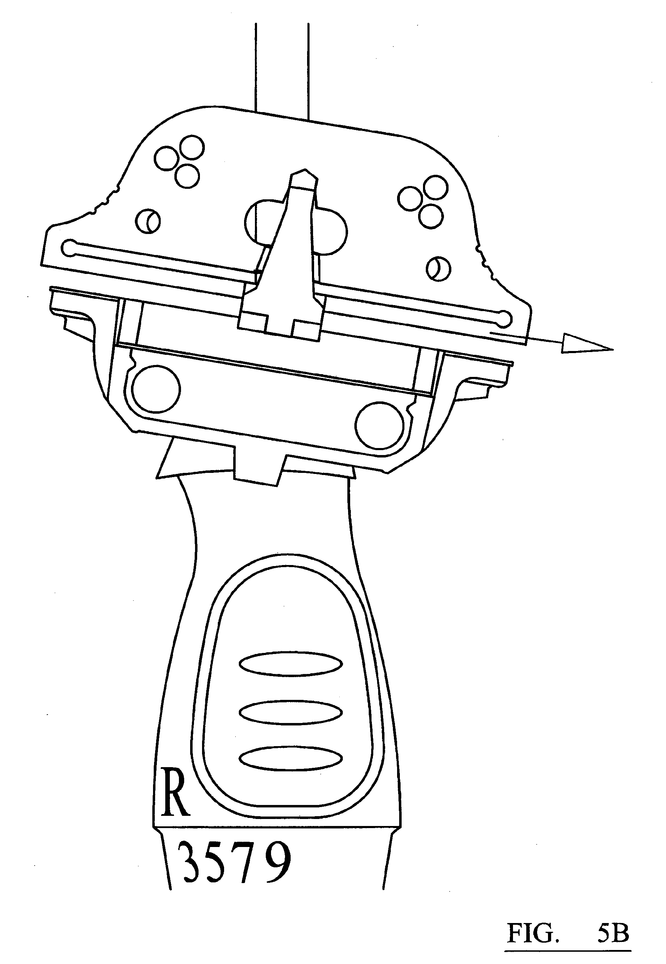

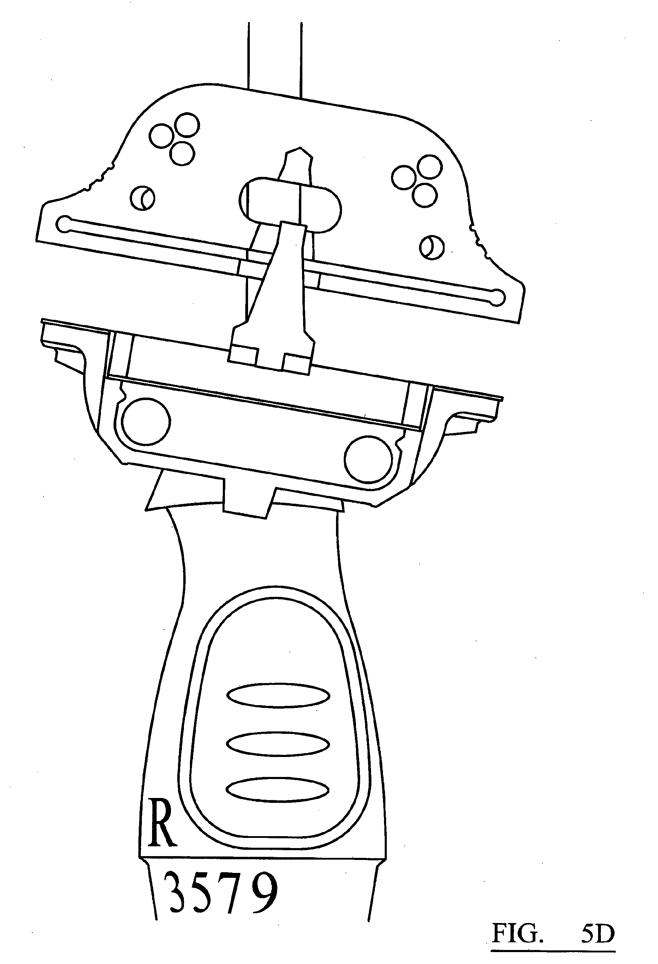

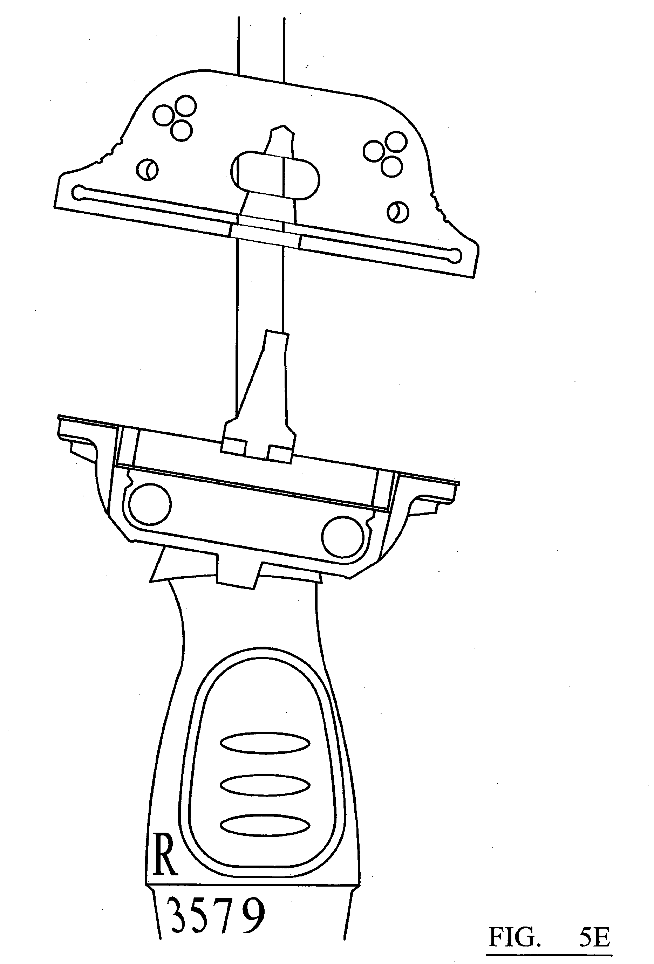

As shown in Figure 5b, in the present system, after relatively little movement, for example as small as 1.5mm, the first 20 and second 22 cylindrical sections of the connection are disengaged. After a further short movement, the first cylindrical section 20 of the attachment protrusion enters the enlarged section 32 of the recess in the cutting guide. At this point, there is significant freedom of movement between the attachment protrusion 18 of the alignment guide and the recess 14 of the cutting guide. As shown in Figure 5d, the disconnection of the cylindrical sections enables simple removal of the attachment protrusion along the anatomical axis defined by cylindrical section 2, without needing complicated manipulation, until as shown in Figure 5e the attachment protrusion 18 is well clear of the cutting guide. The cylindrical section of the rod can then be withdrawn from the intramedullary canal, leaving the cutting guide in place.

Unlike prior art systems, the stepped nature of the attachment protrusion 18 and corresponding recess, including first and second cylindrical portion 20, 22 with different diameters, enables disconnection of an attachment protrusion to be achieved over much shorter distances. This gives greater freedom of movement between the parts, simplifying separation of the alignment guide from the cutting guide after the cutting guide is in place. This can allow a user more freedom in choice of the technique used to disconnect the cutting guide and allow one handed removal in certain circumstances.

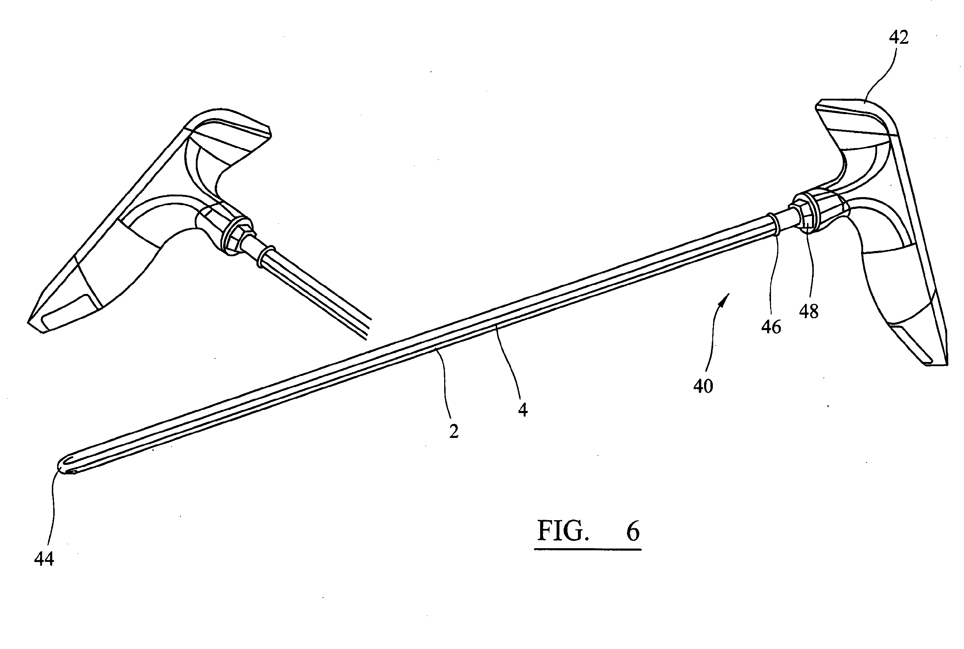

The system is used with an intramedullary rod 40 which is illustrated in its entirety in Figure 6. The intramedullary rod 40 comprises a handle 42, a cylindrical section 2 having grooves 4 formed therein (as described above) and a rounded end 44 at the distal end of the cylindrical section 2, furthest from the handle 42. At a proximal end of the cylindrical section 2, close to the handle 42, a protrusion 46 is provided which extends circumferentially around the cylindrical section 2. Proximal of the protrusion 46, a second protrusion 48 is formed around the longitudinal axis defined by the cylindrical section 2. As will be described in more detail below, protrusion 46 and protrusion 48 form parts of a restraining system for retaining an alignment guide in position on the intramedullary rod 40 while the rod 40 is being inserted or removed from an intramedullary canal.

The protrusion 46 is about 65mm from the proximal end of the handle 42. This distance, and the length of the rod 40, may be varied depending on the length of rod 40 required to extend beyond an alignment guide 6 when engaged with the restraining system. For example, the rod 40 may extend up to 300mm. In use the rod 40 may not be inserted into an intramedullary canal to its full length. The depth of insertion may be limited, for example by a hip stem already present in the canal from an earlier hip replacement procedure. To allow for this, the alignment guide 6 can be released from the restraining system and moved along the rod 42 to engage the bone surface.

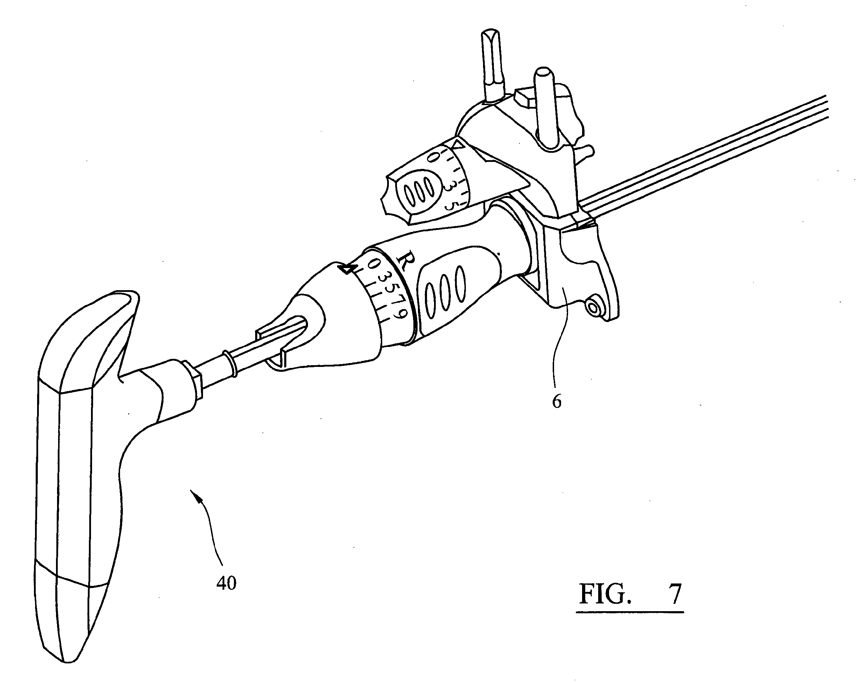

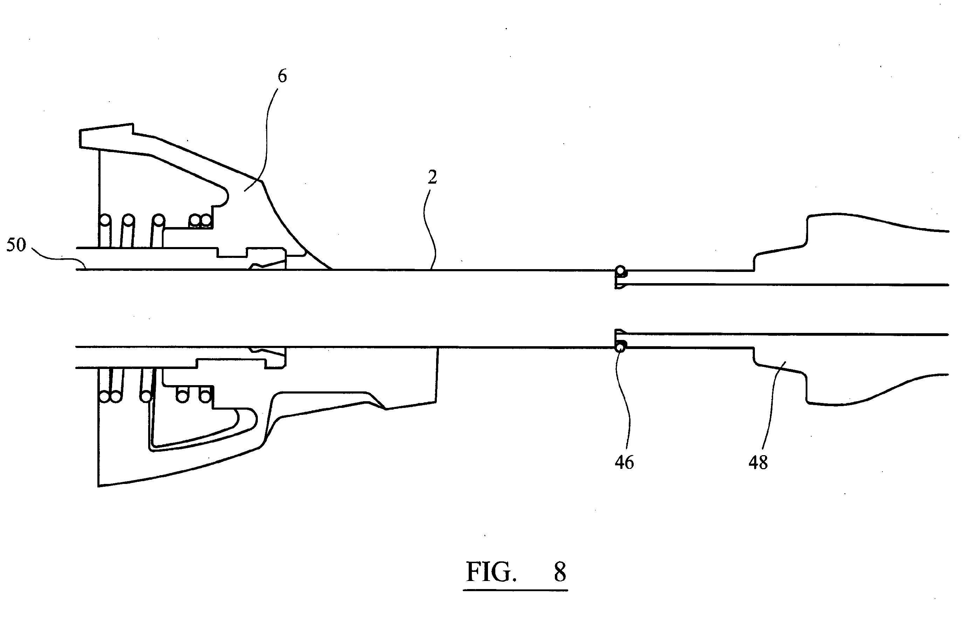

Figure 7 depicts a perspective view of the proximal end of an intramedullary rod 40 with an alignment guide 6 mounted thereon. A cross-section showing the way in which the alignment guide 6 is mounted on the intramedullary rod 40 is given in Figure 8. Figure 8 depicts how the alignment guide 6 comprises a through bore 50 which receives cylindrical section 2 of the intramedullary rod 40. As depicted in Figure 8, the alignment guide 6 can be moved freely along the longitudinal axis relative to the rod and rotated relative to that axis. During insertion and removal of the intramedullary rod to the intramedullary canal, the free movement of the alignment guide can mean that two hands are required, one to insert the rod and the other to ensure that the alignment guide does not move relative to the rod during insertion.

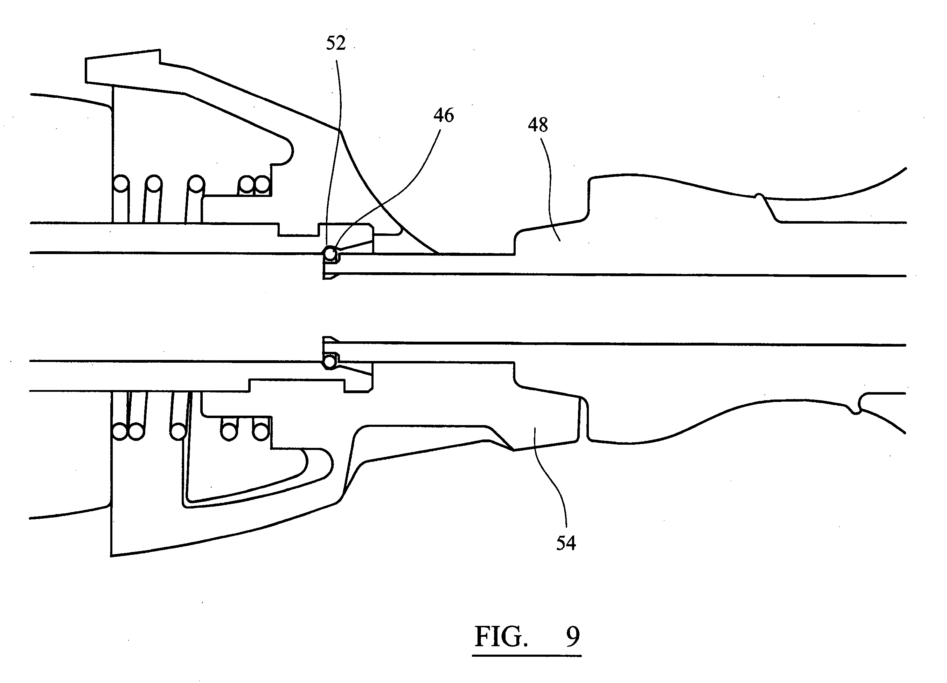

To secure the alignment guide relative to the rod, Figure 9 shows how protrusions 46, 48 engage corresponding features in the alignment guide to prevent longitudinal movement of the alignment guide along the rod and also to prevent rotation of the alignment guide relative to the rod. Ring shaped protrusion 46 on the intramedullary rod is formed from a resilient material. This engages a corresponding groove 52 in the through bore 50 of the alignment guide 6. The resilient nature of the protrusion 46 means that it can be compressed by a small force before expanding into the groove 52. This holds the alignment guide 6 securely on the rod 40, preventing relative longitudinal movement.

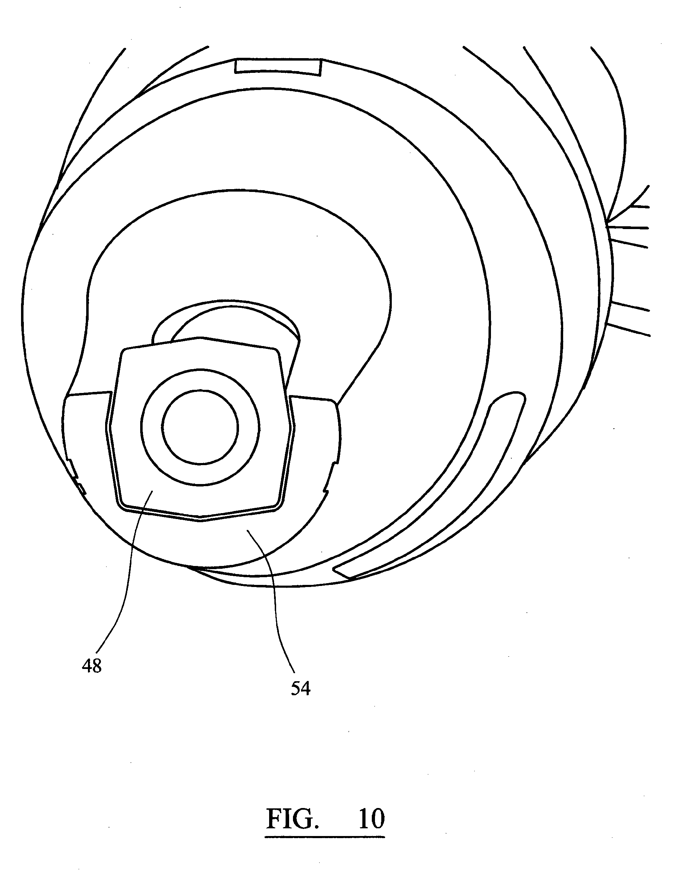

In some embodiments, the protrusion 46 may be made of a material with a high coefficient of friction so that it can also prevent rotation of the alignment guide about the longitudinal axis as well as longitudinal movement. However, a second protrusion 48 may also be provided to prevent rotation. Although not clear from the cross-section in Figure 9, protrusion 48 has a polygonal shape centred on the longitudinal axis. This engages a corresponding recess 54 formed in the alignment guide. Figure 10 shows the engagement between protrusion 48 and recess 54 more clearly. Protrusion 48 has a generally octagonal shape centred on the longitudinal axis. Together, the engagement of protrusion 48 with the recess 54 prevents rotation of the alignment guide 6 relative to the rod 40 when the first protrusion 46 is engaged with groove 52.

Thus, the connection between the rod and the alignment guide can be made secure during insertion or removal of the intramedullary rod. When it is desired to use the alignment guide 6 to place the cutting guide 8 in the correct position, the alignment guide 6 is moved longitudinally in a distal direction to disengage both protrusion 48 from channel 54 and protrusion 46 from groove 52. Alignment guide 6 is then free to translate and rotate about longitudinal axis of the cylindrical section 2.

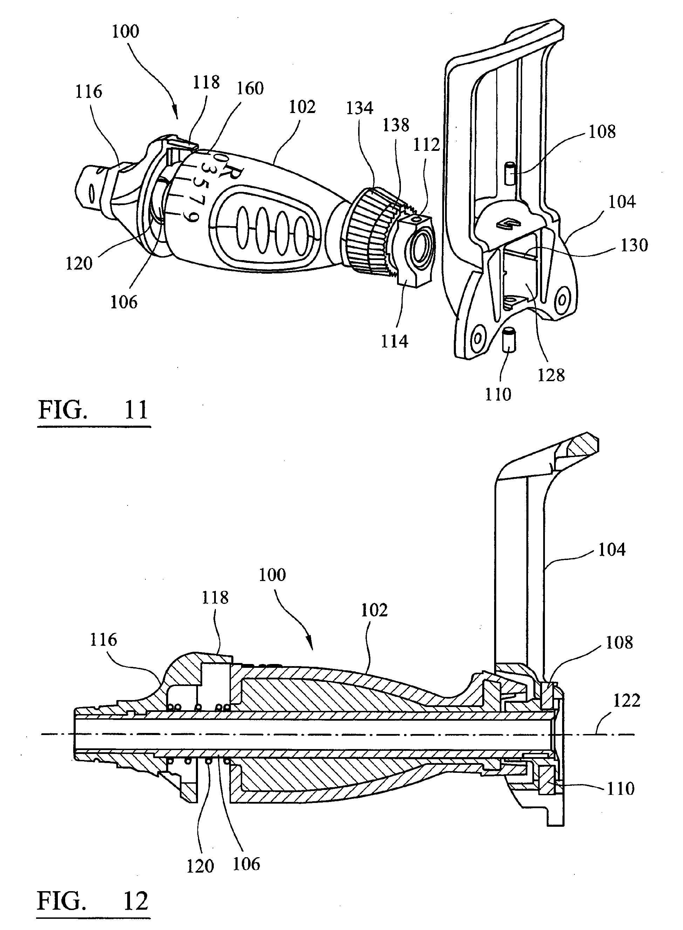

In alternate embodiments (not illustrated) other constructions may be used to provide resilient engagement between a protrusion and a recess. For example, a protrusion may be provided on the intramedullary rod which is not itself resilient. It can be engaged by a recess in the through bore of the alignment guide which adjoins a resilient material, such as an elastomer, on its proximal side. In this construction the proximal end of the recess can deform when the alignment is moved proximally to engage the protrusion in the recess. When the protrusion is engaged, the resilience of the material results in it resuming its original shape and retaining the protrusion in the recess. In another alternate construction, a recess could be provided on the intrameduallary rod and a protrusion provided in the through bore of the alignment guide. The protrusion is resiliently biased into the through bore to engage the recess. The resilient biasing could be provided by use of a resilient material, or by a separate biasing element, such as a spring, acting on the protrusion. Figure 11 depicts a perspective view of an alignment guide 100 which allows a fine degree of control over the angular adjustment. As shown in Figure 11, the alignment guide comprises an adjustment member 102 and a pivoting member 104. The adjustment member is disposed over a longitudinal shaft 106 which defines a longitudinal axis. Longitudinal shaft 106 is hollow, enabling the alignment guide to be installed on an intramedullary rod (not shown). The pivot member 104 is pivotally attached to the shaft 106 by passing pins 108, 110 through openings defined in the pivot member 104 and engaging corresponding openings 112, 114 formed in an end of the shaft 106.

An indicator member 116 is provided at the other end of the shaft to the pivotal connection. This includes a pointer 118 which extends over the end of the adjustment member 102 to overlap a visual indicia of the degree of angular adjustment applied by the adjustment member 102.

The adjustment member 102 is shorter than the distance between the end of indicator member 116 and the pivot point 112, 114. This enables adjustment member 102 to translate back and forth along the longitudinal axis 122. A resilient member 120, which is a helical spring in this embodiment, is disposed around the shaft 106. This provides a force to push the adjustment member 102 towards the pivot point 112, 114 in the absence of an applied force.

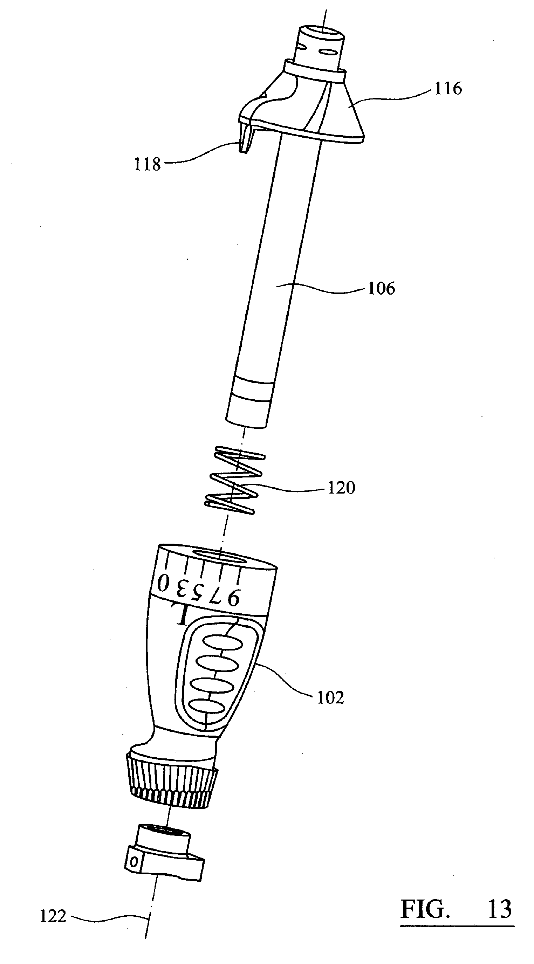

The assembled alignment guide 100 is shown in cross-section in Figure 12. This enables the relationship of the various components to the longitudinal axis 122 to be seen clearly. Figure 13 depicts an exploded diagram showing the construction between the shaft 106, resilient member 120 and adjustment member 102.

Adjustment member 102 includes an end portion which comprises a plurality of pairs of facets 134. Each pair of facets 134 is contiguous with another pair of facets 134. The forward end of the edge between each pair of facets comprises a cut away portion 138. The configuration of the facets 134 and cut away portions 138 will be described in more detail below.

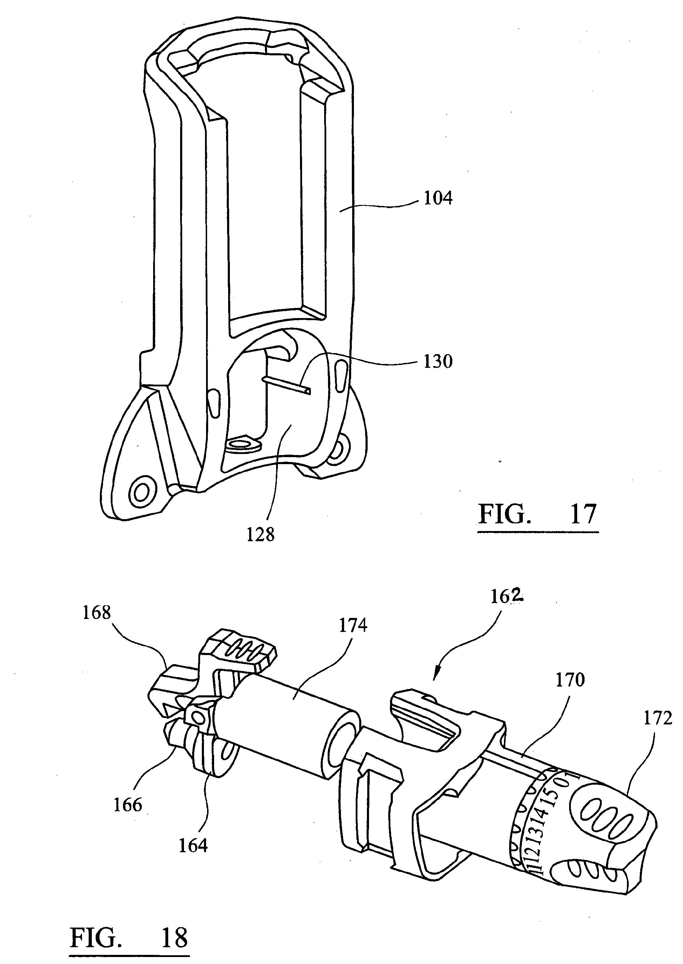

The pivoting member 104 comprises a recess 128 for receiving the end portion of the adjustment member. The recess 128 includes projections 130. The projections 130 are positioned to engage one pair of facets 134 when the end portion of the adjustment member 102 is located in the recess. In the absence of an applied force, the force provided by resilient member 120 ensures that a pair of facets 134 is engaged with the projections 130 of the recess 128. The configuration of the recess 128 and projections 130 can be seen more clearly in Figure 17 which is a perspective view of the pivoting member from the opposite direction to that shown in Figure 11.

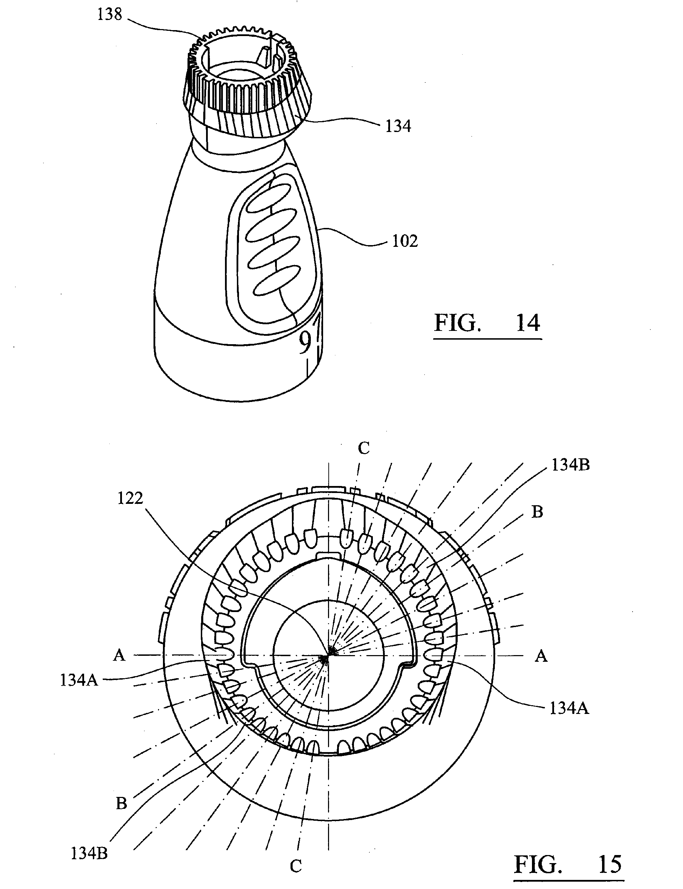

In use, the interaction between a pair of facets 134 on the adjustment member 102 with the projections 130 on the recess 128 acts to rotate the pivoting member about the axis defined by the pins 108, 110. This pivoting is achieved by the specific arrangement of facets 134 provided on the adjustment member 102. The arrangement of these facets will now be described with reference to Figures 14, 15 and 16A-16C.

Figure 14 depicts a perspective view of the adjustment member 102. It shows how the adjustment member comprises a plurality of facets 134 at one end. Facets 134 are arranged in mutually opposed pairs about the longitudinal axis. The configuration of each pair of facets 134 is chosen so that they define an axis which is angled with respect to the longitudinal axis. Figure 15 depicts an end view of the adjustment member 102. It shows how the facets are evenly spaced at regular angular spacings around the longitudinal axis 122. In this embodiment, there are nineteen pairs of facets respectively defining angles of 0° and ±9°.

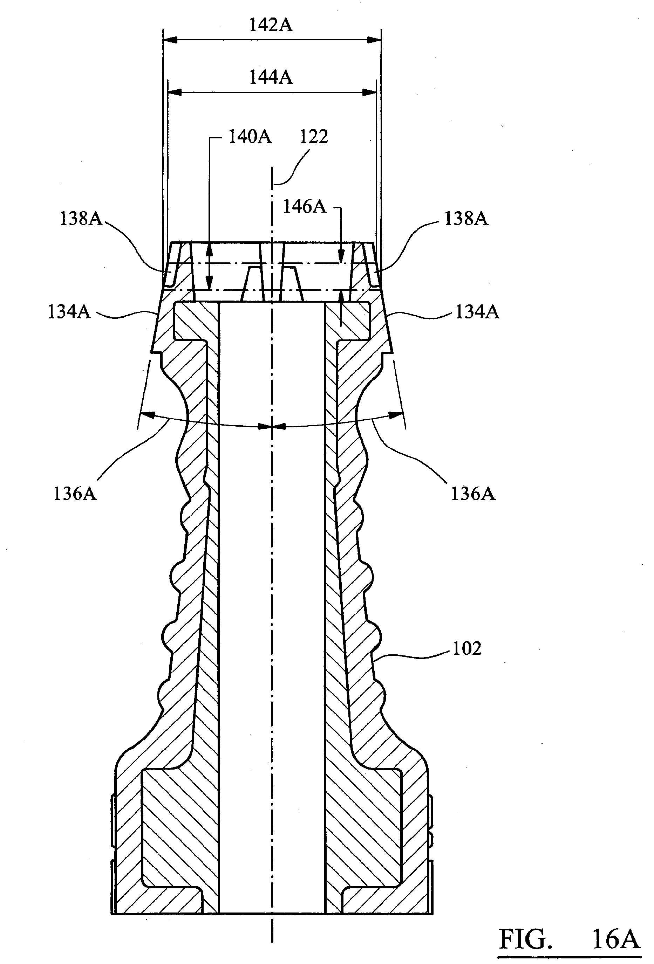

Figure 16A shows a cross-section along line A-A in Figure 15. This pair of facets 134A defines an axis which is coincident with the longitudinal axis 122, or at an angle of 0°. In this example, all of the pairs of facets 134 define a taper of 20°. Thus, both facets 134A are offset by 10° from the longitudinal axis to define a taper of 20°. This angular adjustment is indicated by arrows 136A in Figure 16A.

As discussed above, to facilitate rotation of the adjustment member 102 when it is disengaged from the recess, cut outs 138 are provided at the end. Cut outs 138A depicted in Figures 16A extend approximately 5.5 mm from the end of the adjustment member 102. This is indicated by reference numeral 140A. The width of the adjustment member just before the cut out is approximately 25 mm, indicated by reference number 142A.

Dimension 144A is approximately 24 mm, showing the taper and dimension 146A is approximately 3mm. Other dimensions may be used in other embodiments depending on the particular requirements.

For an angle of 0°, i.e. an axis which is coincident with the longitudinal axis Figure 16A shows that the configuration of the end portion in cross-section at the pair of facets 134A is symmetrical. Thus, when pair of facets 134A engage the projections 130 the recess is rotated to be aligned with the longitudinal axis.

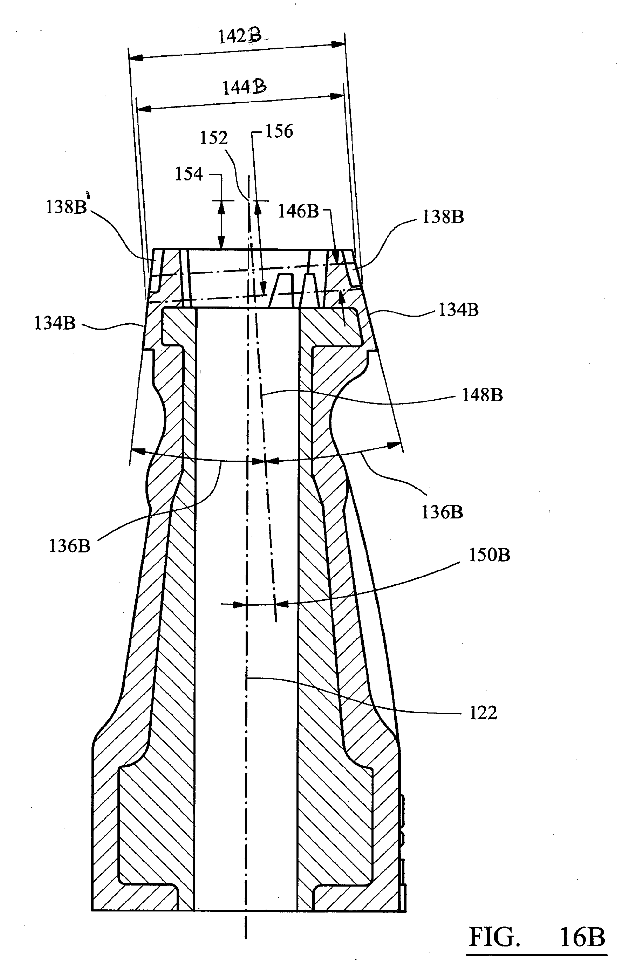

Figure 16B depicts a cross-section along line B-B in Figure 15. At this position, the pair of facets 134B together define an angled axis 148B with respect to the longitudinal axis 122. As indicated by angular dimension 150B, the angled axis 148B is 4° offset from longitudinal axis 122. Angled axis 148B is defined by facets 134B which have been rotated about a point 152. Point 152 lies on the longitudinal axis 122 approximately 5.5 mm from the top of the adjustment member 102, as indicated by distance 154. The taper of the facets 134B is the same as for facets 134A, 20°. However, the taper is defined with reference to the angled axis 148B. This means that when the facets 134B engage the projections 130 the pivoting member will be pivoted through 4° because of the self-centering nature of the taper. Point 152 is chosen to be coincident with the axis of pins 108, 110.

When facets 134B are engaged with pivoting member, the pivoting member is rotated relative to the longitudinal axis consistent with the rotation of the facets 134B along angled axis 148B. Thus, the cut out 138B extends a different distance either side of the adjustment member 102 to ensure that they are the same distance from the pivoting member, when facets 134B are engaged by projections 130. Dimension 156 is approximately 11 mm from the pivot point 152. This 11 mm distance is measured in the direction of angled axis 148B. Thus, cut out 138B is shorter than cut out 138B'. Dimensions 142B, 144B and 146B correspond to dimensions 142A, 144A and 146A for consistency with all embodiments.

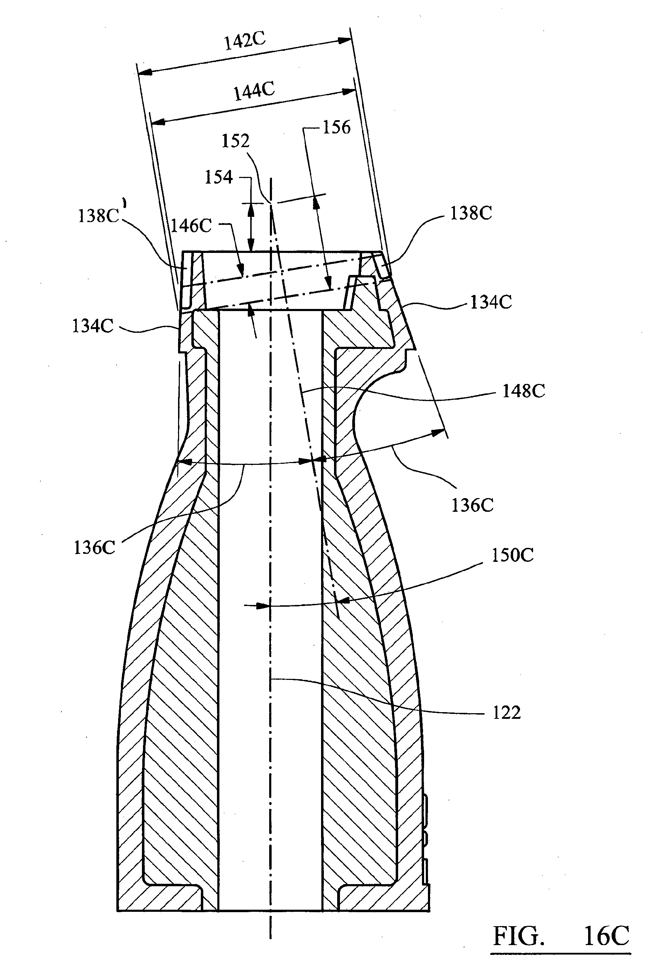

To further assist the explanation, Figure 16C depicts a cross-section of the adjustment member 102 taken along line CC in Figure 15. This corresponds to an adjustment of 9° as indicated by angle 150C in Figure 16C. The angling of angled axis 148C is more pronounced in this cross-section. This means that the distance of cut outs 138C on the right hand side of the diagram is again shorter than the cut out 138C' on the left hand side. The length of the cut out is again determined by projecting a line approximately 11 mm from the pivot point 152 and extending the cutout 138C, 138C' in the direction of the angled axis 148C distance 156 (approximately 11 mm in this embodiment). The remaining dimensions 142C, 144C, 146C remain the same as 142A, 144B and 146C.

Figure 16C demonstrates how the facets 134C are still tapered with respect to the longitudinal axis 122. This is because the taper angle of 20° means that with the 9° relative angle of axis 148C there remains 1° of taper depicted on the left hand side of Figure 16C. This ensures that the taper remains with respect to the longitudinal axis (although it is not symmetrical about the longitudinal axis 122).

In use, the angle of the pivoting member is adjusted by withdrawing the adjustment member 102 proximally against the biassing force of resilient member 120. This disengages the facets from the projections in the pivoting member 104. The adjustment member is then rotated until the indicator 118 points at the desired degree of angular adjustment. This is indicated by markings or indicia 160 on the adjustment member 102. The adjustment member can then be released and the action of the resilient member 120 pushes the end of the adjustment member into the recess 128 of the pivoting member. The pair of facets 134 corresponding to the desired angular adjustment as indicated by indicator 118 engage projections 130. The taper ensures that the pivoting member is centred and securely located on the facets. Depending on the angle of the axis defined by the pair of facets, the pivoting member is turned to the desired angle by the engagement of the facets with the projection.

In this embodiment, a cutting guide is attached to the pivoting member 104 by an intermediate translating assembly 162. Translating assembly 162 comprises an attachment member 164 which includes a stepped connection 166 and clip 168 for attaching a cutting guide (not shown) and a translation adjustment mechanism 170. Translation adjustment mechanism 170 comprises an adjustment dial 172 which adjusts the translation of the cutting guide relative to the alignment guide by adjusting the degree to which shaft 174 is inserted into a corresponding recess in translation adjustment guide 170. (The parts of this assembly are shown in exploded form in Figure 18 for clarity).

It will be appreciated that the configuration depicted in Figures 11-18 differs in some minor aspects of appearance from the configuration depicted in Figures 1-10 and 19. The features of angular adjustment and features of the adjustment member described in relation to Figures 11-18 can be applied to Figures 1-10 and 19.

Where dimensions are described, they are for example only and are not limiting. Alternative dimensions may be used in other embodiments.

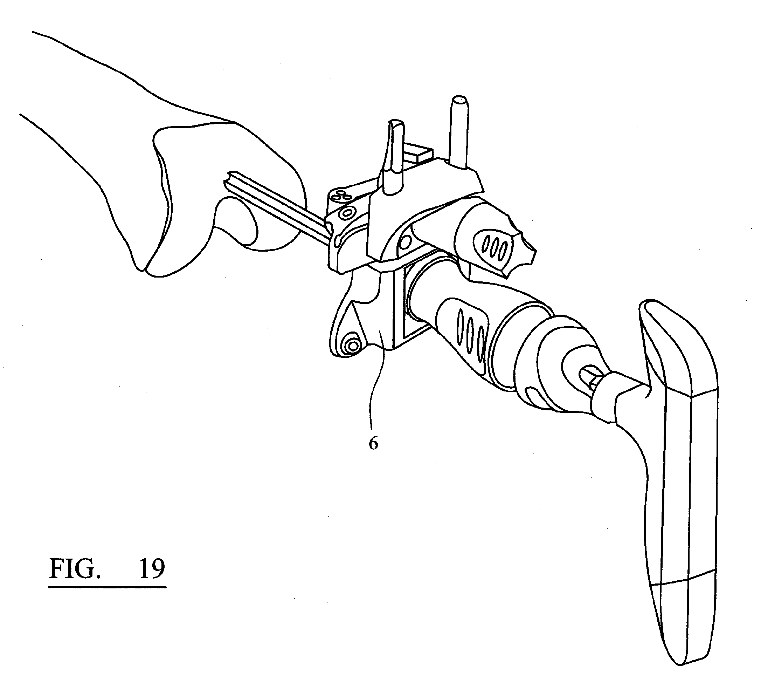

Figure 19 depicts a system of alignment guide, rod, handle and cutting guide in use, inserted into a femur before the alignment guide is advanced along the rod to engage the cutting guide with the femur.

The elements of the above described system are constructed from medical grade materials. For example the rod may be manufactured from medical grade metal and the other components from medical grade plastics materials or metals.

Although a system comprising an alignment guide, cutting guide and rod has been described, the stepped attachment protrusion for connecting the cutting guide and alignment guide can be used in systems which do not include a rod. Likewise the restraining system between the alignment guide and rod can be used in systems which do not include a cutting guide. The stepped attachment system and the restraining system can be used with other alignment guides than the faceted guide described above, for example they may be used with the mechanism discussed in WO-A-2009/037471.

The elements of the above described system are constructed from medical grade materials. For example the rod may be manufactured from medical grade metal and the other components from medical grade plastics materials or metals.

## Claims

1. A surgical instrument system for use in an orthopaedic surgical procedure to implant a knee joint prosthesis, for placing a cutting guide on a patient's bone, comprising:
an intramedullary rod which includes a cylindrical member (2), and which has a handle (42) at its proximal end,
an alignment guide (6) comprising a body section having a through bore (50), which defines a longitudinal axis and which can receive the cylindrical member of the intramedullary rod such that the alignment guide can slide along the rod,
a cutting guide (8) which can be fastened to a patient's bone to guide a cut in a bone cutting step, the alignment guide and the cutting guide being capable of being attached to one another so that the alignment guide can control the orientation of the cutting guide relative to the intramedullary axis, and
a restraining system for retaining the alignment guide towards the proximal end of the rod and preventing relative movement of the alignment guide along the longitudinal axis of the rod,
**characterised in that** the instrument system is for placing the cutting guide on a patient's femur, the restraining system comprising a resiliently deformable element (46) which is provided on one of the rod and the alignment guide, and a recess (52) which is provided on the other of the rod and the alignment guide and configured to engage the resilient element in such a way that it is capable of being released to allow the alignment guide to slide along the rod when the rod has been inserted into the patient's intramedullary cavity, allowing the alignment guide to be attached to the cutting guide while it is in contact with the patient's femur.

2. The surgical instrument system according to claim 1, wherein the resilient member (46) is disposed on the surface of the rod.

3. The surgical instrument system according to claim 1, wherein the recess is a circumferential recess (52) which is formed in the through bore of the alignment guide (6) for engaging the resilient member (46).

4. The surgical instrument system according to any one of the preceding claims, wherein the restraining system is further for preventing rotation of the alignment guide (6) relative to the rod about the longitudinal axis.

5. The surgical instrument system according to claim 4, wherein the restraining system further comprises a protrusion (48) on the rod for engaging a corresponding recess (54) in the alignment guide (6).

6. The surgical instrument system according to claim 5, wherein the protrusion (48) comprises a polygon with a constant cross section along the longitudinal axis.

7. The surgical instrument system according to claim 6, wherein the polygon has rotational symmetry about the longitudinal axis.

## Patentansprüche

1. Chirurgisches Instrumentensystem zur Verwendung bei einem orthopädischen chirurgischen Verfahren zur Implantation einer Kniegelenksprothese zum Platzieren einer Schneidführung auf dem Knochen eines Patienten, umfassend:
einen Marknagel, der ein zylindrisches Element (2) enthält und der einen Griff (42) an seinem proximalen Ende aufweist,
eine Ausrichtungsführung (6) mit einem Körperabschnitt mit einer Durchgangsbohrung (50), die eine Längsachse definiert und die das zylindrische Element des Marknagels aufnehmen kann, sodass die Ausrichtungsführung entlang des Nagels gleiten kann,
eine Schneidführung (8), die an dem Knochen eines Patienten befestigt werden kann, um einen Schnitt in einem Knochenschneideschritt zu führen, wobei die Ausrichtungsführung und die Schneidführung in der Lage sind, aneinander angebracht zu werden, sodass die Ausrichtungsführung die Orientierung der Schneidführung relativ zur Markachse steuern kann, und
ein Rückhaltesystem zum Halten der Ausrichtungsführung in Richtung zum proximalen Ende des Nagels und zum Verhindern einer Relativbewegung der Ausrichtungsführung entlang der Längsachse des Nagels,
**dadurch gekennzeichnet, dass** das Instrumentensystem zum Platzieren der Schneidführung auf dem Femur eines Patienten dient, wobei das Rückhaltesystem ein elastisch verformbares Element (46), das an dem Nagel oder der Ausrichtungsführung vorgesehen ist, und eine Ausnehmung (52) aufweist, die an dem anderen von dem Nagel und der Ausrichtungsführung vorgesehen und konfiguriert ist, um mit dem elastischen Element so in Eingriff zu treten, dass es in der Lage ist, freigegeben zu werden, um zuzulassen, dass die Ausrichtungsführung entlang des Nagels gleitet, wenn der Nagel in den Markkanal des Patienten eingeführt worden ist, wodurch die Ausrichtungsführung an der Schneidführung anbringbar ist, während sie sich in Kontakt mit dem Femur des Patienten befindet.

2. Chirurgisches Instrumentensystem nach Anspruch 1, wobei das elastische Element (46) auf der Oberfläche des Nagels angeordnet ist.

3. Chirurgisches Instrumentensystem nach Anspruch 1, wobei die Ausnehmung eine runde Ausnehmung (52) ist, die in der Durchgangsbohrung der Ausrichtungsführung (6) für einen Eingriff mit dem elastischen Element (46) ausgebildet ist.

4. Chirurgisches Instrumentensystem nach einem der vorangehenden Ansprüche, wobei das Rückhaltesystem ferner zur Verhinderung einer Drehung der Ausrichtungsführung (6) relativ zum Nagel um die Längsachse dient.

5. Chirurgisches Instrumentensystem nach Anspruch 4, wobei das Rückhaltesystem ferner einen Vorsprung (48) an dem Nagel für einen Eingriff mit einer korrespondierenden Ausnehmung (54) in der Ausrichtungsführung (6) umfasst.

6. Chirurgisches Instrumentensystem nach Anspruch 5, wobei der Vorsprung (48) ein Polygon mit einem konstanten Querschnitt entlang der Längsachse umfasst.

7. Chirurgisches Instrumentensystem nach Anspruch 6, wobei das Polygon Rotationssymetrie um die Längsachse aufweist.

## Revendications

1. Système d'instrument chirurgical pour utilisation dans une procédure chirurgicale orthopédique pour implanter une prothèse de l'articulation du genou, pour placer un guide de coupe sur un os d'un patient, comprenant :
une tige intramédullaire qui inclut un élément cylindrique (2) et qui a une poignée (42) à son extrémité proximale,
un guide d'alignement (6) comprenant une section de corps ayant un trou traversant (50) qui définit un axe longitudinal et qui peut recevoir l'élément cylindrique de la tige intramédullaire de telle sorte que le guide d'alignement peut coulisser le long de la tige,
un guide de coupe (8) qui peut être fixé à un os du patient pour guider une coupe lors d'une étape de coupe d'os, le guide d'alignement et le guide de coupe étant aptes à être fixés l'un à l'autre de façon que le guide d'alignement puisse commander l'orientation du guide de coupe relativement à l'axe intramédullaire, et
un système de restriction pour retenir le guide d'alignement vers l'extrémité proximale de la tige et pour prévenir un déplacement relatif du guide d'alignement le long de l'axe longitudinal de la tige,
**caractérisé en ce que** le système d'instrument est destiné au placement d'un guide de coupe sur le fémur d'un patient, le système de restriction comprenant un élément élastiquement déformable (46) qui est réalisé sur l'un parmi la tige et le guide d'alignement, et un évidement (52) qui est réalisé sur l'autre parmi la tige et le guide d'alignement et qui est configuré pour venir en prise avec l'élément résilient de telle sorte qu'il est apte à être relâché pour permettre au guide d'alignement de coulisser le long de la tige lorsque la tige a été insérée dans la cavité intramédullaire du patient, en permettant que le guide d'alignement soit fixé au guide de coupe pendant qu'il est en contact avec le fémur du patient.

2. Système d'instrument chirurgical selon la revendication 1, dans lequel l'élément résilient (46) est disposé sur la surface de la tige.

3. Système d'instrument chirurgical selon la revendication 1, dans lequel l'évidement est un évidement circonférentiel (52) qui est formé dans le trou traversant du guide d'alignement (6) pour la mise en prise avec l'élément résilient (46).

4. Système d'instrument chirurgical selon l'une quelconque des revendications précédentes, dans lequel le système de restriction est prévu en outre pour prévenir une rotation du guide d'alignement (6) relativement à la tige autour de l'axe longitudinal.

5. Système d'instrument chirurgical selon la revendication 4, dans lequel le système de restriction comprend en outre une saillie (48) sur la tige pour l'engagement dans un évidement correspondant (54) dans le guide d'alignement (6).

6. Système d'instrument chirurgical selon la revendication 5, dans lequel la saillie (48) comprend un polygone d'une section transversale constante le long de l'axe longitudinal.

7. Système d'instrument chirurgical selon la revendication 6, dans lequel le polygone a une symétrie de rotation autour de l'axe longitudinal.
